# EUROPÄISCHE PATENTSCHRIFT

(11) **EP 0 312 836 B1**
(45) Veröffentlichungstag und Bekanntmachung des Hinweises auf die Patenterteilung: **24.02.1993**
(21) Anmeldenummer: 88116523.7
(22) Anmeldetag: 06.10.1988
(51) Int. Cl.: C07D 251/34, C08G 18/08, C08G 18/78, C08G 18/80

(54) **In Wasser lösliche oder dispergierbare, blockierte Polyisocyanate, ein Verfahren zu ihrer Herstellung und ihre Verwendung**
Water-soluble or -dispersable, blocked polyisocyanates, a method for their preparation and their use
Polyisocyanates bloqués, solubles ou dispersables dans l'eau, un procédé pour leur préparation et leur utilisation

(30) Priorität: 17.10.1987 DE 3735198
(43) Veröffentlichungstag der Anmeldung: 26.04.1989
(73) Patentinhaber: BAYER AG, 51368 Leverkusen (DE)
(72) Erfinder: Schäfer, Walter, Dr., D-5653 Leichlingen (DE); Müller, Hanns Peter, Dr., D-5060 Bergisch-Gladbach 2 (DE); Dhein, Rolf, Dr., D-4150 Krefeld (DE)

(56) Entgegenhaltungen:
- EP-A- 0 000 963
- DE-A- 185 184
- US-A- 4 491 663

## Beschreibung

Die vorliegende Erfindung betrifft die Verwendung von neuartigen, in Wasser löslichen bzw. dispergierbaren, blockierten Polyisocyanaten, die als die Löslichkeit bzw. Dispergierbarkeit in Wasser gewährleistende Gruppierung Cyanharnstoff-Anionen in chemisch gebundener Form aufweisen, als Härter in Zweikomponenten-Lacken.

In Wasser lösliche oder dispergierbare, blockierte Polyisocyanate sind beispielsweise aus der DE-A-24 56 469 bekannt, Diese Verbindungen können in wäßriger Lösung bzw. Dispersion in Kombination mit wasserlöslichen oder -dispergierbaren Polyhydroxylverbindungen als Bindemittelkomponente für aus wäßriger Phase zu verarbeitende Polyurethanlacke verwendet werden.

In Zweikomponenten-Polyurethanlacken stellen die als Bindemittelkomponente verwendeten "Lackpolyisocyanate" den im allgemeinen vergleichsweise niedermolekularen Härter für die höhermolekularen Polyhydroxylverbindungen dar, wobei dieser Härter im Interesse einer einwandfreien, zu hochwertigen Überzügen führenden Vernetzung eine mittlere Funktionalität von mindestens 2,5, vorzugsweise von mindestens 3, aufweisen sollte.

Beim Verfahren zur Herstellung der bekannten, in Wasser löslichen bzw. dispergierbaren, blockierten Polyisocyanate wird jedoch ein nicht unwesentlicher Teil der in den Ausgangspolyisocyanaten vorliegenden Isocyanatgruppen durch die hydrophile Modifizierung verbraucht (Umsetzung eines Teils der Isocyanatgruppen mit der hydrophilen Modifizierungskomponente). Dadurch wird das Vernetzungsvermögen der Polyisocyanate stark reduziert.

Es war daher die der Erfindung zugrunde liegende Aufgabe, neue Zweikomponenten-Systeme auf Basis von blockierten Polyisocyanaten zur Verfügung zu stellen, die in Wasser lösliche bzw. dispergierbare Polyisocyanate enthalten, bei denen die die Löslichkeit bzw. Dispergierbarkeit bewirkende Gruppe an der Vernetzungsreaktion teilnimmt, so daß durch den Einbau der hydrophilen Gruppierungen in die Ausgangspolyisocyanate deren Vernetzungspotential nicht reduziert wird.

Diese Aufgabe konnte mit der nachstehend näher beschriebenen erfindungsgemäßen Verwendung von speziellen, blockierten Polyisocyanaten gelöst werden. Die in den erfindungsgemäß zu verwendenden Polyisocyanaten vorliegenden hydrophilen Zentren weisen eine Doppelfunktion auf, d.h., sie bewirken nicht nur die Löslichkeit bzw. Dispergierbarkeit der Polyisocyanate in Wasser, sondern nehmen auch an der Vernetzungsreaktion bei der erfindungsgemäßen Verwendung der blockierten Polyisocyanate teil, wobei sie gleichzeitig ihre hydrophile Eigenschaft verlieren.

Die EP-A-0 185 184 beschreibt zwar bereits anionische Cyanharnstoffgruppen aufweisende Polyisocyanate, die diese charakteristische anionische Struktureinheit aufweisen, bei deren Herstellung teilblockierte Polyisocyanate als Ausgangsmaterialien eingesetzt worden sind. Die Vorveröffentlichung lehrt jedoch an keiner Stelle, daß das ganz spezielle Vernetzungsprinzip (Seite 14, Zeile 12 bis Seite 15, Zeile 9 der Vorveröffentlichung) mit der bekannten, chemisch völlig andersartigen Vernetzung von Polyhydroxylverbindungen mit blockierten Polyisocyanaten in Form der erfindungsgemäßen blockierten Polyisocyanate sinnvoll kombiniert werden kann.

Gegenstand der Erfindung ist die Verwendung von blockierten Polyisocyanaten in in Wasser gelöster bzw. dispergierter Form in Kombination mit wäßrigen Lösungen bzw. Dispersionen von organischen Verbindungen mit mindestens zwei gegenüber Isocyanatgruppen reaktionsfähigen Wasserstoffatomen, gegebenenfalls unter gleichzeitiger Mitverwendung von weiteren Hilfs- und Zusatzmitteln zur Herstellung von Flächengebilden durch Beschichtung geeigneter Substrate mittels der kombinierten wäßrigen Lösungen bzw. Dispersionen, Entfernung des Wassers und gleichzeitiger oder anschließender thermischer Vernetzung der so erhaltenen Beschichtung, dadurch gekennzeichnet, daß man als blockierte Isocyanate solche verwendet, die
a) im statistischen Mittel pro Molekül mindestens eine Struktureinheit der allgemeinen Formel

   B-CO-NH-

   und
b) Struktureinheiten der 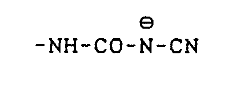 Formelin einer die Löslichkeit bzw. Dispergierbarkeit in Wasser gewährleistenden Menge aufweisen,
wobei im statistischen Mittel pro Molekül insgesamt mindestens zwei Struktureinheiten der unter a) und b) genannten Formeln vorliegen, und wobei B für einen Rest steht, wie er durch Entfernung des aciden Wasserstoffatoms aus einem monofunktionellen Blockierungsmittel für organische Isocyanate erhalten wird.

Polyisocyanate, die zur Herstellung der erfindungsgemäß zu verwendenden blockierten Polyisocyanate als Ausgangsmaterialien eingesetzt werden können, sind beispielsweise:
(i) einfache organische Polyisocyanate des Molekulargewichtsbereichs 168 bis 300 wie z.B. 1,6-Diisocyanatohexan, 1-Isocyanato-3,3,5-trimethyl-5-isocyanatomethyl-cyclohexan, 2,4- und/oder 2,6-Diisocyanatotoluol, 4,4'- und/oder 2,4'-Diisocyanatodiphenylmethan oder 4,4'-Diisocyanato-dicyclohexylmethan.
   Diese einfachen Diisocyanate werden allerdings weniger bevorzugt verwendet.
(ii) Modifizierte "Lackpolyisocyanate" mit einem mittleren Molekulargewicht von bis 1000, d.h., die an sich bekannten Biuret-, Isocyanurat-, Uretdion- oder Oxadiazintriongruppen aufweisen, modifizierten Polyisocyanate auf Basis der unter (i) genannten einfachen Diisocyanate, wie beispielsweise das Tris-(6-isocyanatohexyl)-biuret oder dessen Gemische mit seinen höheren Homologen oder das Tris-(6-isocyanatohexyl)-isocyanurat oder seine Gemische mit seinen höheren Homologen und gegebenenfalls mit dem Bis-(6-isocyanatohexyl)-uretdion oder Bis-(6-isocyanatohexyl)-oxadiazintrion.

Vorzugsweise werden Ausgangspolyisocyanate mit aliphatisch gebundenen Isocyanatgruppen eingesetzt. Aliphatische Lackpolyisocyanate der unter (ii) beispielhaft genannten Art sind besonders bevorzugte Ausgangsmaterialien.

Grundsätzlich ist es auch möglich, bei der Herstellung der Cyanharnstoffaniongruppen enthaltenden blockierten Polyisocyanate ionisch modifizierte teilblockierte Polyisocyanate der aus der Chemie der Polyurethandispersionen an sich bekannten Art mitzuverwenden, um zu Polyisocyanat-Additionsprodukten zu gelangen, die außer den erfindungswesentlichen anionischen Struktureinheiten der obengenannten allgemeinen Formel noch eingebaute Carboxylat- oder Sulfonatgruppen aufweisen, Dies wäre beispielsweise dadurch möglich, daß man teilblockierte NCO-Prepolymere einsetzt, die unter Verwendung bzw. Mitverwendung von Carboxylat- oder Sulfonatgruppen aufweisenden mehrwertigen Alkoholen oder unter Verwendung der entsprechenden Hydroxycarbonsäure bzw. Hydroxysulfonsäuren unter anschließender Neutralisation der Säuregruppen hergestellt worden sind. Die Mitverwendung derartiger anionischer Aufbaukomponenten ist jedoch weniger bevorzugt, da einerseits die Hydrophilie der erfindungswesentlichen anionischen Struktureinheiten für die Löslichkeit bzw. Dispergierbarkeit der Polyisocyanat-Additionsprodukte im allgemeinen ausreicht, und da andererseits bei Mitverwendung derartiger anionischer Aufbaukomponenten die eingebauten anionischen Gruppen in den letztendlich erhaltenen Flächengebilden weiterhin vorliegen würden und nichts zur Vernetzung beitragen, was dem Zweck der Erfindung zuwiderlaufen würde.

Besonders bevorzugt werden als Ausgangspolyisocyanate die unter (ii) beispielhaft genannten Lackpolyisocyanate, insbesondere die bekannten Biuret-Polyisocyanate oder Isocyanurat-Polyisocyanate auf Basis von 1,6-Diisocyanatohexan verwendet.

Geeignete Blockierungsmittel B-H zur Herstellung der blockierten Polyisocyanate sind insbesondere Verbindungen mit einer gegenüber Isocyanatgruppen reaktionsfähigen Gruppe, die mit organischen Isocyanaten zwischen 20 und 120°C eine Additionsreaktion eingehen und deren so erhaltene Additionsprodukte im Gemisch mit nichtflüchtigen primäre Hydroxylgruppen aufweisenden Polyolen bei Temperaturen zwischen 100 und 200°C unter Freisetzung des Blockierungsmittels mit den nicht-flüchtigen Polyolen unter Urethanbildung reagieren. Geeignete derartige Blockierungsmittel sind z.B. Lactame, wie z.B. ε-Caprolactam, δ-Valerolactam, γ-Butyrolactam; Oxime, wie z.B. Formaldoxim, Acetaldoxim, Methylethylketonoxim, Cyclohexanonoxim, Acetophenonoxim, Benzophenonoxim oder Diethylglyoxim; C-H-acide Verbindungen, wie z.B. Malonsäuredialkylester, Acetylaceton, Acetessigsäurealkylester; Phenole, wie z.B. Phenol, o-Methylphenol; Imide, wie z.B. Phthalimid, Imidazol oder Triazol; sekundäre oder tertiäre Alkohole, wie z.B. Isopropanol oder tert. Butanol. Vorzugsweise werden Lactame, Oxime, Azole und C-H-acide Verbindungen eingesetzt.

Die erste Stufe der Herstellung der erfindungsgemäß zu verwendenden Polyisocyanate besteht in einer an sich bekannten Teilblockierung der Isocyanatgruppen des Ausgangspolyisocyanats. Die Menge an Blockierungsmittel wird hierbei so bemessen, daß in dem resultierenden, teilblockierten Polyisocyanat neben, für die weitere Modifizierungsreaktion erforderlichen, freien Isocyanatgruppen im statistischen Mittel pro Molekül mindestens eine, vorzugsweise mindestens zwei, blockierte Isocyanatgruppen vorliegen. Im statistischen Mittel müssen in den teilblockierten Polyisocyanaten insgesamt mindestens zwei freie und blockierte Isocyanatgruppen vorliegen. Vorzugsweise liegen in den teilblockierten Polyisocyanaten insgesamt 2,8 bis 6, insbesondere 3 bis 4, freie und blockierte Isocyanatgruppen vor. Für die im zweiten Reaktionsschritt durchzuführende hydrophile Modifizierung der teilblockierten Polyisocyanate ist es im allgemeinen ausreichend, wenn der Gehalt der teilblockierten Polyisocyanate an freien Isocyanatgruppen bei 0,5 bis 3,5 Milliäquivalenten/g liegt. Bei der Durchführung des zweiten Reaktionsschritts müssen jedoch Art und Mengenverhältnisse der Reaktionspartner so gewählt werden, daß in den resultierenden anionischen, blockierten Polyisocyanaten, ohne Berücksichtigung des gegebenenfalls ebenfalls blockierte Isocyanatgruppen aufweisenden Kations, pro Molekül im statistischen Mittel mindestens eine blockierte Isocyanatgruppe und insgesamt mindestens 2 blockierte Isocyanat- und anionische Cyanharnstoffgruppen vorliegen. Vorzugsweise liegt die Summe der beiden Gruppen im statistischen Mittel bei 2,8 bis 6, insbesondere bei 3 bis 4 pro Molekül, wobei, besonders bevorzugt, mindestens 2 dieser Gruppen blockierte Isocyanatgruppen darstellen. Grundsätzlich ist es nicht erforderlich bei der 2. Stufe des Verfahrens, bezogen auf die freien NCO-Gruppen, äquivalente Mengen des Cyanamid-Salzes einzusetzen, da evtl. verbleibende freie Isocyanatgruppen spätestens bei der Überführung der Umsetzungsprodukte in eine wäßrige Lösung oder Dispersion die freien NCO-Gruppen mit dem Wasser unter Kettenverlängerung abreagieren, was der Verwendbarkeit der Produkt nicht entgegensteht. In der 2. Stufe liegt demzufolge das Äquivalentverhältnis von NCO-Gruppen zu Cyanharnstoff-Salz im allgemeinen bei 1:1 bis 1,5:1.

Die Blockierungsreaktion wird im allgemeinen bei 20 bis 120°C in Abwesenheit eines Lösungsmittels durchgeführt. Je nach Art des Blockierungsmittels empfiehlt sich die Mitverwendung eines Katalysators. Bei Verwendung von Hydroxylgruppen aufweisenden Blockierungsmitteln empfiehlt sich die Verwendung eines Metallkatalysators, wie z.B. Dibutylzinndilaurat; bei Verwendung von Blockierungsmitteln mit aktivierten Methylengruppen empfiehlt sich die Verwendung von basischen Katalysatoren, wie z.B. Diazabicyclooctan, Triethylamin, Alkalialkoholate oder Alkaliphenolate, wie z.B. Natriumphenolat. Die Katalysatoren werden, falls überhaupt, in Mengen von 0,05 bis 0,5 Gew.-%, bezogen auf Gesamtreaktionsansatz, eingesetzt.

In der zweiten Stufe der Herstellung der blockierten Polyisocyanate werden die verbleibenden Isocyanatgruppen des teilblockierten Polyisocyanats mit einem Ammoniumsalz des Cyanamids zum entsprechenden Cyanharnstoffsalz umgesetzt. Die Umsetzung verläuft schematisch nach folgender Gleichung: 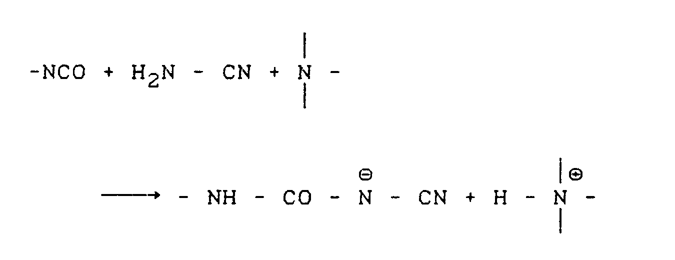
Bei dieser Umsetzung werden die Reaktionspartner vorzugsweise in stöchiometrischen Mengen miteinander zur Reaktion gebracht, wobei die Reaktionstemperatur im allgemeinen bei -10 bis +40°C, vorzugsweise bei 0 bis 35°C, liegt. Die Salze des Cyanamids können hierbei als solche eingesetzt oder auch in situ durch gleichzeitige Verwendung von Cyanamid und dem entsprechenden tert. Amin hergestellt werden. Die Herstellung von geeigneten Ammoniumsalzen des Cyanamids kann beispielsweise dergestalt erfolgen, daß man die Base mit dem Cyanamid im Temperaturbereich von -10 bis 35°C, vorzugsweise 0 bis 25°C, in organischem oder wäßrigem Milieu (geeignete Lösungsmittel wären beispielsweise Aceton oder Tetrahydrofuran) miteinander zur Reaktion bringt, wobei die Menge der Base so bemessen wird, daß für jedes Mol an Cyanamid 0,5 bis 2 Mol, vorzugsweise 1 Mol basische Stickstoffatome zur Verfügung stehen. Bei der Herstellung der Salze in situ werden analoge Mengenverhältnisse der Base und des Cyanamids eingesetzt.

Bei den den Salzen zugrunde liegenden Basen handelt es sich um Ammoniak oder um tertiäre Amine, deren tertiäre Aminstickstoffatome vorzugsweise ausschließlich mit aliphatischen Kohlenstoffatomen verknüpft sind. Die tert. Amine können eine oder auch mehrere derartige tertiäre Aminogruppen aufweisen. Die bevorzugten tertiären Amine weisen ein Molekulargewicht von 59 bis 1000, vorzugsweise 59 bis 200 auf. In Betracht kommen insbesondere aliphatische tertiäre Amine, die gegebenenfalls Hydroxylgruppen als Substituenten aufweisen. Hierzu gehören beispielsweise Trimethylamin, Triethylamin, Tripropylamin, N,N-Dimethyl-stearylamin, N,N-Dimethylaminoethanol, N-Methyl-diethanolamin oder Triethanolamin. Grundsätzlich ist es jedoch auch möglich, solche Ammoniumsalze des Cyanamids zu verwenden, deren Ammoniumgruppen auf tertiären Aminen basieren, die Urethan-, Allophanat-, Harnstoff-, Biuret-, Isocyanurat-, Uretdion-, Uretonimin-, Ester-und/oder Ethergruppen aufweisen, und insbesondere solche, die neben mindestens einer tertiären Aminogruppe mindestens zwei blockierte Isocyanatgruppen der allgemeinen Formel

B′-CO-NH-

tragen, wobei B′ bezüglich seiner Bedeutung der Definition von B entspricht, jedoch von B verschieden sein kann.

Derartige, modifizierte tert. Amine können beispielsweise durch Umsetzung von Aminoalkoholen der beispielhaft genannten Art mit organischen Polyisocyanaten der oben unter (i) beispielhaft genannten Art (Einbau von Urethangruppen) oder mit Allophanat-, Harnstoff-, Biuret-, Isocyanurat-, Uretdion-, Uretonimin-, Ester- und/oder Ethergruppen aufweisenden NCO-Prepolymeren erhalten werden. Tert. Amine, die neben mindestens einer tert. Aminogruppe mindestens zwei blockierte Isocyanatgruppen enthalten, entstehen beispielsweise durch Umsetzung eines teilblockierten Polyisocyanats der oben beispielhaft genannten Art, welche mindestens zwei blockierte Isocyanatgruppen und mindestens eine freie Isocyanatgruppe aufweist, mit Aminoalkoholen der oben beispielhaft genannten Art oder auch mit tertiären Aminen, die neben der tertiären Aminogruppe mindestens eine primäre und/oder sekundäre Aminogruppe aufweisen. Zu letzteren Verbindungen gehören beispielsweise N,N-Dimethylethylendiamin, N,N-Dimethylpropylendiamin-1,3 oder N,N-Bis-(2-aminopropyl)-methylamin.

Zur Herstellung von tertiären Aminen, die neben den tert. Aminogruppen blockierte Isocyanatgruppen aufweisen, kann beispielsweise so vorgegangen werden, daß das Ausgangspolyisocyanat, beispielsweise ein Biuret-Polyisocyanat auf Basis von 1,6-Diisocyanatohexan oder ein Isocyanuratgruppen aufweisendes Polyisocyanat auf Basis des gleichen Diisocyanats mit einem hydroxy- oder aminofunktionellen tert. Amin der genannten Art bei 0 bis 120°C in Substanz oder in einem organischen Lösungsmittel (z.B. Toluol, Tetrahydrofuran, Methyl-ethylketon, Aceton) unter Verwendung von solchen Mengenverhältnissen der Ausgangsverbindungen zur Reaktion gebracht wird, daß in dem resultierenden, tert. Aminogruppen aufweisenden Polyisocyanat im statistischen Mittel noch mindestens zwei freie Isocyanatgruppen vorliegen, wonach die noch vorliegendenen freien Isocyanatgruppen bei 25 bis 120°C mit einer stöchiometrischen Menge des Blockierungsmittels B'-H umgesetzt werden.

Die zweite Reaktionsstufe der Herstellung der erfindungsgemäß zu verwendenden Polyisocyanate, d.h. die Umsetzung der teilblockierten Polyisocyanate mit den Salzen des Cyanamids bzw. mit Cyanamid und der entsprechenden Base (Herstellung der Salze in situ) kann in Substanz oder auch in Gegenwart eines geeigneten Lösungsmittels erfolgen. Vorzugsweise werden mit Wasser unbegrenzt mischbare Lösungsmittel eines unterhalb 100°C liegenden Siedepunktes wie z.B. Aceton, Methylethylketon oder Tetrahydrofuran mitverwendet.

Nach erfolgter Umsetzung kann das Verfahrensprodukt durch Einrühren in Wasser und evtl. nachfolgendes Abdestillieren des Lösungsmittels in die wäßrige Phase als Lösung oder als Dispersion überführt werden. Bei der Überführung der so hergestellten, erfindungsgemaß zu verwendenden Polyisocyanate in ihre wäßrige Lösung bzw. Dispersion werden im allgemeinen solche Mengen an Wasser eingesetzt, daß die Lösungen bzw. Dispersionen einen Feststoffgehalt von 30 bis 70 Gew.-% aufweisen.

Falls bei der Herstellung der erfindungsgemäß zu verwendenden Polyisocyanate Lösungsmittel der zuletzt beispielhaft genannten Art mitverwendet worden sind, können diese im Anschluß an die Herstellung der wäßrigen Lösung bzw. Dispersion gewünschtenfalls destillativ entfernt werden. Hierbei erweist es sich als vorteilhaft, daß die erfindungsgemäß zu verwendenden Polyisocyanate im pH-Bereich von 8 bis 9 auch bei höheren Temperaturen, wie sie bei destillativen Abtrennung von Lösungsmittel erreicht werden, hydrolysestabil sind.
Die so erhaltenen wäßrigen Lösungen oder Dispersionen der erfindungsgemäß zu verwendenden Verbindungen stellen wertvolle Bindemittelkomponenten, d.h. Härter für wäßrige Beschichtungsmittel, dar. Die erfindungsgemäß zu verwendenden Verbindungen eignen sich insbesondere als Härter für in Wasser gelöste bzw. dispergierte organische Verbindungen, die mindestens zwei gegenüber Isocyanatgruppen reaktionsfähige Wasserstoffatome aufweisen. Hierzu gehören beispielsweise die bekannten wäßrigen Polyurethandispersionen, die beispielsweise in Form der Urethangruppen aktive Wasserstoffatome aufweisen, wäßrige Polyacrylatdispersionen mit gegenüber Isocyanatgruppen reaktionsfähigen Wasserstoffatomen, in Wasser gelöste niedermolekulare Polyamine mit mindestens zwei primären und/oder sekundären Aminogruppen, in Wasser gelöste bzw. dispergierte Alkydharze mit gegenüber Isocyanatgruppen reaktionsfähigen Gruppen, in Wasser gelöste oder dispergierte organische Polyhydroxylverbindungen, insbesondere in Wasser gelöst oder dispergiert vorliegende Polyester- oder Polyetherpolyole der aus der Polyurethanchemie an sich bekannten Art. Auch Gemische unterschiedlicher derartiger Verbindungen mit gegenüber Isocyanatgruppen reaktionsfähigen Wasserstoffatomen können als Reaktionspartner für die blockierten Polyisocyanate zur Herstellung von wäßrigen Beschichtungsmitteln zum Einsatz gelangen.

Bei der erfindungsgemäßen Verwendung der blockierten Polyisocyanate können die üblichen Hilfs- und Zusatzmittel der Lacktechnologie wie beispielsweise Pigmente, Verlaufshilfsmittel oder Füllstoffe mitverwendet werden. Es ist ebenfalls möglich, neben den erfindungswesentlichen, blockierten Polyisocyanaten in den Beschichtungsmitteln weitere Vernetzer, beispielsweise reaktive Carbonylverbindungen, niedermolekulare N-Methylolverbindungen, Aminoplast-oder Phenoplast-Vorkondensate mitzuverwenden. Die Gesamtmenge der Vernetzer kann hierbei in weiten Grenzen schwanken, sie muß jedoch so gewählt werden, daß der letztendlich erreichte Vernetzungsgrad den Anforderungen an die Beschichtung bezüglich Härte, Wasser- und Lösungsmittelresistenz, sowie mechanischer Eigenschaften genügt.

Die wäßrigen Beschichtungsmittel, die die erfindungswesentlichen, blockierten Polyisocyanate als wesentliche Härterkomponente enthalten, können nach allen beliebigen Methoden der Beschichtungstechnologie auf beliebige, hitzeresistente Substrate aufgetragen werden. Nach erfolgter Applikation werden die Beschichtungen im allgemeinen innerhalb des Temperaturbereichs von 100 bis 200°C, vorzugsweise 120 bis 180°C, ausgehärtet, wobei zunächst das Wasser und die gegebenenfalls vorliegenden, flüchtigen tert. Amine verdampfen.

Der wesentliche Vorteil der erfindungsgemäß zu verwendenden blockierten Polyisocyanate gegenüber den hydrophil-modifizierten, blockierten Polyisocyanaten des Standes der Technik ist nun in dem Umstand begründet, daß bei der Hitzebehandlung während der Aushärtung nicht nur eine Reaktion zwischen blockierten Isocyanatgruppen und gegenüber Isocyanatgruppen reaktionsfähigen Gruppen, sondern außerdem eine Kondensationsreaktion der anionischen Cyanharnstoff-Gruppierung unter Abspaltung des tert. Amins nach folgendem Schema erfolgt: 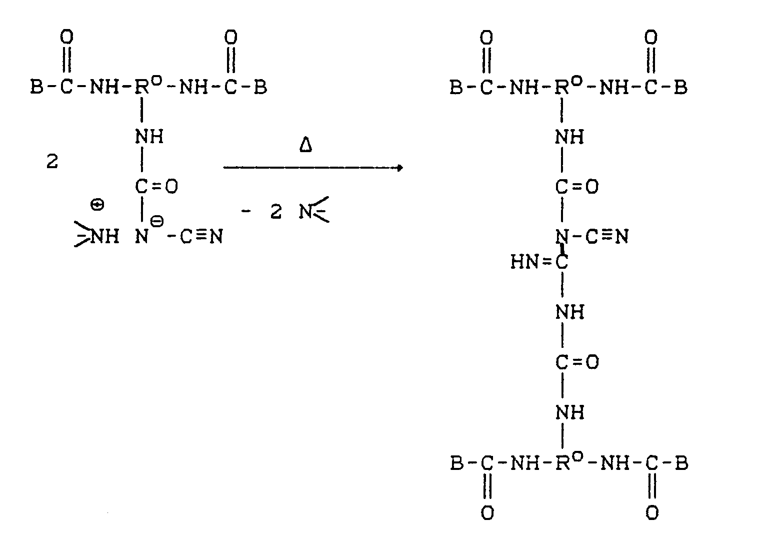
In diesen Formeln steht B für den indifferenten Rest des Blockierungsmittels B-H und R^{o} für den indifferenten Rest eines als Ausgangspolyisocyanat eingesetzten Triisocyanats.

Gemäß dem genannten Reaktionsschema entsteht somit aus dem modifizierten Triisocyanat, welches zwei blockierte Isocyanatgruppen und eine anionische Cyanharnstoffgruppe aufweist, ein blockiertes Tetraisocyanat, welches gegenüber den Verbindungen mit gegenüber Isocyanatgruppen reaktionsfähigen Wasserstoffatomen als tetrafunktioneller Vernetzer wirkt. Je nach Abspalttemperatur des Blockierungsmittels B-H ist es jedoch auch denkbar, daß während der Vernetzungsreaktion bei der thermischen Aushärtung der Beschichtung zunächst eine Reaktion zwischen blockierten Isocyanatgruppen und den gegenüber Isocyanatgruppen reaktionsfähigen Gruppen stattfindet und die weitere Vernetzungsreaktion gemäß dem obengenannten Schema erst im Anschluß hieran erfolgt. Ebenfalls denkbar ist selbstverständlich ein gleichzeitiges Ablaufen der beiden Vernetzungsreaktionen. Da in allen diesen Fällen die zunächst vorliegende anionische Gruppierung unter Verlust ihrer hydrophilen Eigenschaften an der Vernetzungsreaktion teilnimmt, rechtfertigt sich die eingangs getroffene Feststellung, daß die anionische Modifizierung der erfindungswesentlichen, blockierten Polyisocyanate im Unterschied zur Modifizierung der bekannten, hydrophil-modifizierten blockierten Polyisocyanate des Standes der Technik keine Beeinträchtigung des Vernetzunggspotentials der blockierten Polyisocyanate bewirkt.

Im Falle der erfindungswesentlichen, blockierten Polyisocyanate auf Basis von flüchtigen tert. Aminen entweichen die tertiären Amine während des Vernetzungsvorgangs. Im Falle der erfindungswesentlichen, blockierten Polyisocyanate auf Basis von schwer-flüchtigen tert. Aminen, verbleiben diese während des Vernetzungsvorgangs im letztendlich erhaltenen Lackfilm und können dort beispielsweise die Wirkung eines Weichmachers ausüben. Im Falle der erfindungswesentlichen, blockierten Polyisocyanate, in denen als Kationen solche auf Basis von tert. Aminen mit mindestens zwei blockierten Isocyanatgruppen vorliegen, erfolgt während der thermischen Aushärtung ebenfalls eine Abspaltung des Amins, welches jedoch dann seinerseits als blockiertes Polyisocyanat an der Vernetzungsreaktion teilnimmt.

In den nachfolgenden Beispielen beziehen sich alle Prozentangaben auf Gewichtsprozente.

### Beispiel 1

Zu 400 g (2 Mol NCO) eines Isocyanuratgruppen aufweisenden Polyisocyanats auf Basis von 1,6-Diisocyanatohexan mit einem Gehalt an freiem 1,6-Diisocyanatohexan von unter 0,7 % werden 116,6 g (1,34 Mol) Butanonoxim und 0,2 g Zinn-II-octoat gegeben und auf 100°C erhitzt. Nach 2 Stunden wird der NCO-Gehalt titrimetrisch bestimmt (NCO-Gehalt = 5,5 %).

515 g (0,67 Mol NCO) des teilblockierten Polyisocyanats werden in 250 g Aceton gelöst. Die so erhaltene Lösung wird anschließend innerhalb eines Zeitraums von 90 Min. bei 10 bis 20°C zu einer Lösung von 28,1 g (0,67 Mol) Cyanamid und 59,6 g (0,67 Mol) N,N-Dimethylaminoethanol in 50 g Aceton unter Rühren zugetropft. Nachdem im IR-Spektrum die Isocyanatbande bei 2260 cm⁻¹ verschwunden ist, werden 600 g Wasser zugegeben. Durch die klare Lösung wird bei 60 bis 70°C so lange Stickstoff geleitet, bis im Destillat kein Aceton mehr festzustellen ist.

Die Lösung hat einen Feststoffgehalt von 50 % und einen Gehalt an blockierten Isocyanatgruppen (berechnet als NCO) von 4,7 %. Der gelöste Feststoff enthält 1,1 Milliäquivalente/g an anionischen Cyanharnstoffgruppen.

### Beispiel 2

Zu 200 g (1 Mol NCO) des Isocyanuratgruppen aufweisenden Polyisocyanats gemäß Beispiel 1 werden 75,7 g (0,67 Mol) ε-Caprolactam und 0,1 g Zinn-II-octoat gegeben und auf 120°C erhitzt. Nach 2 Stunden wird der NCO-Gehalt titrimetrisch bestimmt (NCO-Gehalt = 5 %).

250 g (0,3 Mol NCO) des teilblockierten Polyisocyanats werden in 100 g Tetrahydrofuran gelöst und zu einer Mischung aus 12,5 g (0,3 Mol) Cyanamid und 26,5 g (0,3 Mol) Dimethylaminoethanol in 20 g Tetrahydrofuran als Lösungsmittel bei 10 bis 20°C innerhalb von 20 Min. zugetropft. Nachdem im IR-Spektrum die Isocyanatbande bei 2260 cm⁻¹ verschwunden ist, werden 250 g Wasser zugegeben. Durch die klare Lösung wird bei 80°C so lange Stickstoff geleitet, bis im Destillat kein Tetrahydrofuran mehr festzustellen ist.

Die Lösung weist bei einem Feststoffgehalt von 53 % einen Gehalt an blockierten Isocyanatgruppen (berechnet als NCO) von 4,7 % auf. Der dispergierte Feststoff enthält 1,0 Milliäquivalente/g an anionischen Cyanharnstoffgruppen.

### Beispiel 3 (Verwendung)

100 g einer wäßrigen Lösung mit einem Feststoffgehalt von 31 % eines mit Ammoniak neutralisierten Polyhydroxypolyacrylats auf Basis von Hydroxyethylmethacrylat, Styrol, Methylmethacrylat, n-Butylacrylat und Acrylsäure im Gew.-Verhältnis 4:2:2:2:1 werden mit 100 g der Lösung aus Beispiel 1 vermischt. Es entsteht eine stabile, etwas milchige Lösung.

Nach Applikation auf ein unbehandeltes Stahlblech entsteht nach 30 Min. bei 150 bei 160°C eine glänzende, gut haftende Beschichtung, die nach 16 Stunden in Wasser oder nach 5 Min. in Aceton keine Erweichung zeigt.

## Patentansprüche

1. Verwendung von blockierten Polyisocyanaten in in Wasser gelöster bzw.dispergierter Form in Kombination mit wäßrigen Lösungen bzw. Dispersionen von organischen Verbindungen mit mindestens Zwei gegenüber Isocyanatgruppen reaktionsfähigen Wasserstoffatomen, gegebenenfalls unter gleichzeitiger Mitverwendung von weiteren Hilfs- und Zusatzmitteln zur Herstellung von Flächengebilden durch Beschichtung geeigneter Substrate mittels der kombinierten wäßrigen Lösungen bzw. Dispersionen, Entfernung des Wassers und gleichzeitiger oder anschließender thermischer Vernetzung der so erhaltenen Beschichtung, dadurch gekennzeichnet, daß man als blockierte Polyisocyanate solche verwendet, die
a) im statistischen Mittel pro Molekül mindestens eine Struktureinheit der allgemeinen Formel
B-CO-NH-
und
b) Struktureinheiten der Formel 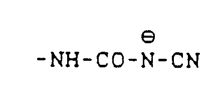 in einer die Löslichkeit bzw. Dispergierbarkeit in Wasser gewährleistenden Menge aufweisen,
wobei im statistischen Mittel pro Molekül insgesamt mindestens zwei Struktureinheiten der unter a) und b) genannten Formeln vorliegen, und wobei B für einen Rest steht, wie er durch Entfernung des aciden Wasserstoffatoms aus einem monofunktionellen Blockierungsmittel für organische Isocyanate erhalten wird.

2. Verwendung gemäß Anspruch 1, dadurch gekennzeichnet daß die blockierten Polyisocyanate
a) im statistischen Mittel pro Molekül mindestens zwei blockierte Isocyanatgruppen
und
b) 0,3 bis 3,5 Milliäquivalente/g an anionischen Struktureinheiten aufweisen.

3. Verwendung gemäß Anspruch 1 und 2, dadurch gekennzeichnet, daß die Gegenionen zu den anionischen Struktureinheiten b) Ammoniumgruppen darstellen, wie sie durch Anlagerung eines Protons an ein tertiäres Amin erhalten werden.

## Claims

1. The use of blocked polyisocyanates dissolved or dispersed in water in combination with aqueous solutions or dispersions of organic compounds containing at least two isocyanate-reactive hydrogen atoms, optionally together with other auxiliaries and additives, for the production of sheet-form materials by coating suitable substrates with the combined aqueous solutions or dispersions, removing the water and simultaneously or subsequently crosslinking the coating obtained by heating, characterized in that the blocked isocyanates used are those which,
a) on a statistical average, contain per molecule at least one structural unit corresponding to the general formula
B-CO-NH-
and
b) structural units corresponding to the formula 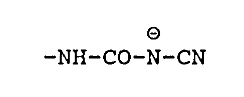 in a quantity which guarantees solubility or dispersibility in water,
a total of at least two structural units corresponding to the formulae shown under a) and b) being present per molecule on a statistical average,
B representing a function of the type obtained by removal of the acidic hydrogen atom from a monofunctional blocking agent for organic isocyanates.

2. The use claimed in claim 1, characterized in that the blocked polyisocyanates contain
a) on a statistical average at least two blocked isocyanate groups per molecule
and
b) 0.3 to 3.5 milliequivalents/g of anionic structural units.

3. The use claimed in claims 1 and 2, characterized in that the counter ions to the anionic structural units b) are ammonium groups obtained by addition of a proton onto a tertiary amine.

## Revendications

1. Utilisation de polyisocyanates protégés sous forme dissoute ou dispersée dans l'eau en association avec des solutions ou dispersions aqueuses de composés organiques portant au moins deux atomes d'hydrogène aptes à réagir vis-à-vis de groupes isocyanato, le cas échéant avec utilisation simultanée d'autres substances auxiliaires et additifs pour la production de corps plans par revêtement de substrats appropriés au moyen des solutions ou dispersions aqueuses associées, élimination de l'eau et réticulation thermique simultanée ou subséquente du revêtement ainsi obtenu, caractérisée en ce qu'on utilise comme polyisocyanates protégés des polyisocyanates qui présentent
a) en moyenne statistique, par molécule, au moins un motif structural de formule générale
B-CO-NH-
et
b) des motifs structuraux de formule 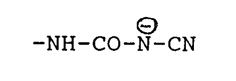 en une quantité garantissant la solubilité ou la dispersibilité dans l'eau,
chaque molécule présentant en moyenne statistique au total au moins deux motifs structuraux des formules mentionnées en a) et b) et B représentant un reste tel qu'on en obtient par l'enlèvement de l'atome acide d'hydrogène d'un agent protecteur monofonctionnel pour isocyanates organiques.

2. Utilisation suivant la revendication 1, caractérisée en ce que les polyisocyanates protégés présentent
a) en moyenne statistique, par molécule, au moins deux groupes isocyanato protégés
et
b) 0,3 à 3,5 milliéquivalents/g de motifs structuraux anioniques.

3. Utilisation suivant les revendications 1 et 2, caractérisée en ce que les ions complémentaires relatifs aux motifs structuraux anioniques b) représentent des groupes ammonium tels qu'on en obtient par addition d'un proton sur une amine tertiaire.
